# EUROPEAN PATENT APPLICATION

(11) **EP 3 539 499 A1**
(43) Date of publication of application: **18.09.2019**
(21) Application number: 19163201.7
(22) Date of filing: 15.03.2019
(51) Int. Cl.: A61B 34/20, A61M 29/02

(54) **NAVIGATION INSTRUMENT WITH OBLIQUELY ORIENTED SENSING COIL**

(30) Priority: 16.03.2018 US 201815923164
(71) Applicant: Acclarent, Inc., Irvine, CA 92618 (US)
(72) Inventor: SHAMELI, Ehsan, Irvine, CA 92618 (US)
(74) Representative: Small, Gary James

(57) **Abstract**

An apparatus includes an elongate body and a sensor. The elongate body has a proximal end and a distal end. The distal end is sized and configured to fit through a naturally occurring orifice of a human body. The sensor is positioned at the distal end of the elongate body. The sensor includes an electrically conductive coil. The coil extends along a longitudinal axis. The coil has a distal terminal winding and a proximal terminal winding. The distal terminal winding extends along a plane that is obliquely oriented relative to the longitudinal axis. The proximal terminal winding extends along a plane that is obliquely oriented relative to the longitudinal axis.

## Description

### BACKGROUND

In some instances, it may be desirable to dilate an anatomical passageway in a patient. This may include dilation of ostia of paranasal sinuses (e.g., to treat sinusitis), dilation of the larynx, dilation of the Eustachian tube, dilation of other passageways within the ear, nose, or throat, etc. One method of dilating anatomical passageways includes using a guide wire and catheter to position an inflatable balloon within the anatomical passageway, then inflating the balloon with a fluid (e.g., saline) to dilate the anatomical passageway. For instance, the expandable balloon may be positioned within an ostium at a paranasal sinus and then be inflated, to thereby dilate the ostium by remodeling the bone adjacent to the ostium, without requiring incision of the mucosa or removal of any bone. The dilated ostium may then allow for improved drainage from and ventilation of the affected paranasal sinus. A system that may be used to perform such procedures may be provided in accordance with the teachings of U.S. Pub. No. 2011/0004057, entitled "Systems and Methods for Transnasal Dilation of Passageways in the Ear, Nose or Throat," published January 6, 2011, the disclosure of which is incorporated by reference herein. An example of such a system is the Relieva® Spin Balloon Sinuplasty™ System by Acclarent, Inc. of Irvine, California.

Image-guided surgery (IGS) is a technique where a computer is used to obtain a real-time correlation of the location of an instrument that has been inserted into a patient's body to a set of preoperatively obtained images (e.g., a CT or MRI scan, 3-D map, etc.), such that the computer system may superimpose the current location of the instrument on the preoperatively obtained images. In some IGS procedures, a digital tomographic scan (e.g., CT or MRI, 3-D map, etc.) of the operative field is obtained prior to surgery. A specially programmed computer is then used to convert the digital tomographic scan data into a digital map. During surgery, special instruments having sensors (e.g., electromagnetic coils that emit alternating current electromagnetic fields and/or are responsive to externally generated alternating current electromagnetic fields) mounted thereon are used to perform the procedure while the sensors send data to the computer indicating the current position of each surgical instrument. The computer correlates the data it receives from the instrument-mounted sensors with the digital map that was created from the preoperative tomographic scan. The tomographic scan images are displayed on a video monitor along with an indicator (e.g., crosshairs or an illuminated dot, etc.) showing the real-time position of each surgical instrument relative to the anatomical structures shown in the scan images. In this manner, the surgeon is able to know the precise position of each sensor-equipped instrument by viewing the video monitor even if the surgeon is unable to directly visualize the instrument itself at its current location within the body.

An example of an electromagnetic IGS systems that may be used in ENT and sinus surgery is the CARTO® 3 System by Biosense-Webster, Inc., of Irvine, California. When applied to functional endoscopic sinus surgery (FESS), balloon sinuplasty, and/or other ENT procedures, the use of IGS systems allows the surgeon to achieve more precise movement and positioning of the surgical instruments than can be achieved by viewing through an endoscope alone. As a result, IGS systems may be particularly useful during performance of FESS, balloon sinuplasty, and/or other ENT procedures where anatomical landmarks are not present or are difficult to visualize endoscopically.

Navigation of the three-dimensional views of the areas surrounding the operative field (e.g., rotating or moving a viewpoint within three-dimensional space) may be accomplished via interaction with an interface device, such as a keyboard or mouse, of an IGS system. These types of interface devices might not be intended for use in a sterile environment, and therefore may not located within reach of a clinician that is performing a medical procedure with the assistance of an IGS system. As a result, clinicians may need to relay navigation instructions to an assistant in another room or area, who will then use the interface device to provide the three-dimensional views that the clinician desires. This process can be time consuming and error prone.

While several systems and methods have been made and used in ENT procedures, it is believed that no one prior to the inventors has made or used the invention described in the appended claims.

### BRIEF DESCRIPTION OF THE DRAWINGS

While the specification concludes with claims which particularly point out and distinctly claim the invention, it is believed the present invention will be better understood from the following description of certain examples taken in conjunction with the accompanying drawings, in which like reference numerals identify the same elements and in which:
FIG. 1A depicts a perspective view of an exemplary dilation instrument assembly, with a guidewire in a proximal position, and with a dilation catheter in a proximal position;
FIG. 1B depicts a perspective view of the dilation instrument assembly of FIG. 1A, with the guidewire in a distal position, and with the dilation catheter in the proximal position;
FIG. 1C depicts a perspective view of the dilation instrument assembly of FIG. 1A, with the guidewire in a distal position, with the dilation catheter in a distal position, and with a dilator of the dilation catheter in a non-dilated state;
FIG. 1D depicts a perspective view of the dilation instrument assembly of FIG. 1A, with the guidewire in a distal position, with the dilation catheter in the distal position, and with a dilator of the dilation catheter in a dilated state;
FIG. 2 depicts a schematic view of an exemplary sinus surgery navigation system being used on a patient seated in an exemplary medical procedure chair;
FIG. 3 depicts a side schematic view of a distal end portion of a navigation guidewire of the system of FIG. 2;
FIG. 4 depicts a side elevational view of an exemplary sensor coil that may be incorporated into the navigation guidewire of FIG. 3; and
FIG. 5 depicts a side elevational view of another exemplary sensor coil that may be incorporated into the navigation guidewire of FIG. 3.

The drawings are not intended to be limiting in any way, and it is contemplated that various embodiments of the invention may be carried out in a variety of other ways, including those not necessarily depicted in the drawings. The accompanying drawings incorporated in and forming a part of the specification illustrate several aspects of the present invention, and together with the description serve to explain the principles of the invention; it being understood, however, that this invention is not limited to the precise arrangements shown.

### DETAILED DESCRIPTION

The following description of certain examples of the invention should not be used to limit the scope of the present invention. Other examples, features, aspects, embodiments, and advantages of the invention will become apparent to those skilled in the art from the following description, which is by way of illustration, one of the best modes contemplated for carrying out the invention. As will be realized, the invention is capable of other different and obvious aspects, all without departing from the invention. Accordingly, the drawings and descriptions should be regarded as illustrative in nature and not restrictive.

It will be appreciated that the terms "proximal" and "distal" are used herein with reference to a clinician gripping a handpiece assembly. Thus, an end effector is distal with respect to the more proximal handpiece assembly. It will be further appreciated that, for convenience and clarity, spatial terms such as "top" and "bottom" also are used herein with respect to the clinician gripping the handpiece assembly. However, surgical instruments are used in many orientations and positions, and these terms are not intended to be limiting and absolute.

It is further understood that any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

### I. Exemplary Dilation Catheter System

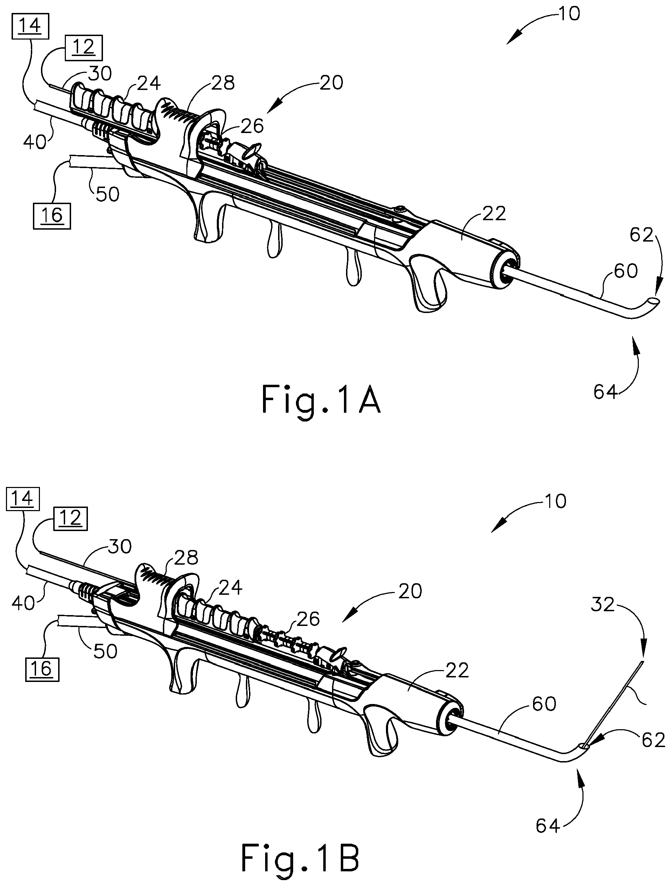

FIGS. 1A-1D show an exemplary dilation instrument assembly (10) that may be used to dilate the ostium of a paranasal sinus; to dilate some other passageway associated with drainage of a paranasal sinus; to dilate a Eustachian tube; or to dilate some other anatomical passageway (e.g., within the ear, nose, or throat, etc.). Dilation instrument assembly (10) of this example comprises a guidewire power source (12), an inflation source (14), an irrigation fluid source (16), and a dilation instrument (20). In some versions, guidewire power source (12) comprises a source of light. In some other versions, guidewire power source (12) is part of an IGS system as described below. Inflation source (14) may comprise a source of saline or any other suitable source of fluid. Irrigation fluid source (16) may comprise a source of saline or any other suitable source of fluid. Again, though, any other suitable source of fluid may be used. It should also be understood that irrigation fluid source (16) may be omitted in some versions.

Dilation instrument (20) of the present example comprise a handle body (22) with a guidewire slider (24), a guidewire spinner (26), and a dilation catheter slider (28). Handle body (22) is sized and configured to be gripped by a single hand of a human operator. Sliders (24, 28) and spinner (26) are also positioned and configured to be manipulated by the same hand that grasps handle body (22).

A guide catheter (60) extends distally from handle body (22). Guide catheter (60) includes an open distal end (62) and a bend (64) formed proximal to open distal end (62). Dilation instrument (20) is configured to removably receive several different kinds of guide catheters (60), each guide catheter (60) having a different angle formed by bend (64). Guide catheter (60) of the present example is formed of a rigid material (e.g., rigid metal and/or rigid plastic, etc.), such that guide catheter (60) maintains a consistent configuration of bend (64) during use of dilation instrument (20). In some versions, dilation instrument (20), is further configured to enable rotation of guide catheter (60), relative to handle body (22), about the longitudinal axis of the straight proximal portion of guide catheter (60), thereby further promoting access to various anatomical structures.

A guidewire (30) is coaxially disposed in guide catheter (60). Guidewire slider (24) is secured to guidewire (30). Translation of guidewire slider (24) relative to handle body (22) from a proximal position (FIG. 1A) to a distal position (FIG. 1B) causes corresponding translation of guidewire (30) relative to handle body (22) from a proximal position (FIG. 1A) to a distal position (FIG. 1B). When guidewire (30) is in a distal position, a distal portion of guidewire (30) protrudes distally from open distal end (62) of guide catheter (60). Guidewire spinner (26) is operable to rotate guidewire (30) about the longitudinal axis of guidewire (30). Guidewire spinner (26) is coupled with guidewire slider (24) such that guidewire spinner (26) translates longitudinally with guidewire slider (24). By way of example only, guidewire (30) may be configured in accordance with at least some of the teachings of U.S. Pat. No. 9,155,492, the disclosure of which is incorporated by reference herein. Other features and operabilities that may be incorporated into guidewire (30) will be apparent to those of ordinary skill in the art in view of the teachings herein.

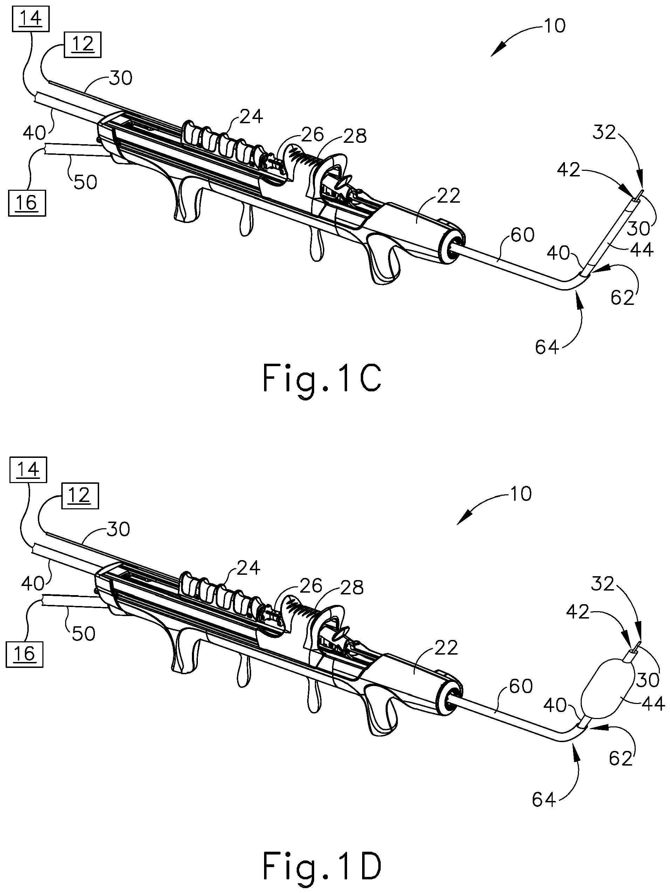

A dilation catheter (40) is coaxially disposed in guide catheter (60). Dilation catheter slider (28) is secured to dilation catheter (40). Translation of dilation catheter slider (28) relative to handle body (22) from a proximal position (FIG. 1B) to a distal position (FIG. 1C) causes corresponding translation of dilation catheter (40) relative to handle body (22) from a proximal position (FIG. 1B) to a distal position (FIG. 1C). When dilation catheter (40) is in a distal position, a distal portion of dilation catheter (40) protrudes distally from open distal end (62) of guide catheter (60). Dilation catheter (40) of the present example comprises a non-extensible balloon (44) located just proximal to open distal end (42) of dilation catheter (40). Balloon (44) is in fluid communication with inflation source (14). Inflation source (14) is configured to communicate fluid (e.g., saline, etc.) to and from balloon (44) to thereby transition balloon (44) between a non-inflated state and an inflated state. FIG. 1C shows balloon (44) in a non-inflated state. FIG. 1D shows balloon (44) in an inflated state. In the non-inflated state, balloon (44) is configured to be inserted into a constricted anatomical passageway. In the inflated state, balloon (44) is configured to dilate the anatomical passageway in which balloon (44) is inserted. Other features and operabilities that may be incorporated into dilation catheter (40) will be apparent to those of ordinary skill in the art in view of the teachings herein.

### II. Exemplary Image Guided Surgery Navigation System

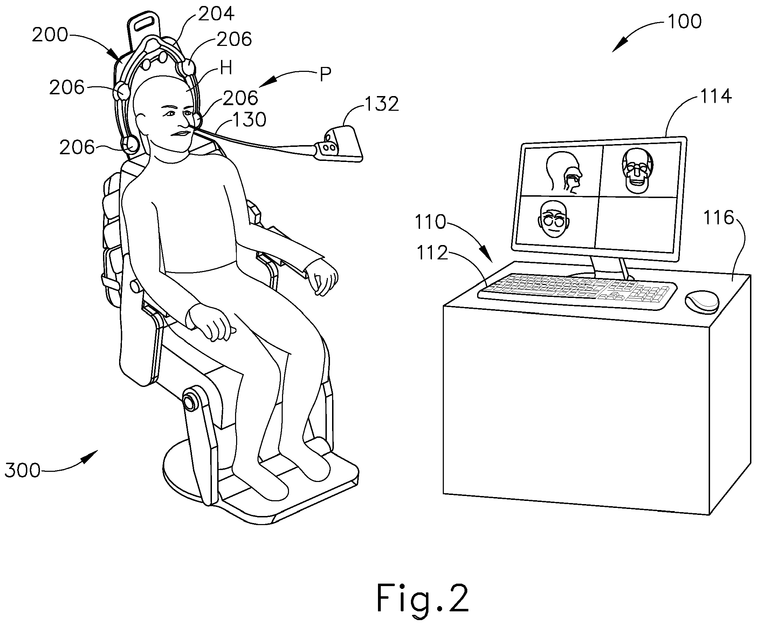

FIG. 2 shows an exemplary IGS navigation system (100) enabling an ENT procedure to be performed using image guidance. In some instances, IGS navigation system (100) is used during a procedure where dilation instrument assembly (10) is used to dilate the ostium of a paranasal sinus; or to dilate some other anatomical passageway (e.g., within the ear, nose, or throat, etc.). In addition to or in lieu of having the components and operability described herein IGS navigation system (100) may be constructed and operable in accordance with at least some of the teachings of U.S. Pat. No. 8,702,626, entitled "Guidewires for Performing Image Guided Procedures," issued April 22, 2014, the disclosure of which is incorporated by reference herein; U.S. Pat. No. 8,320,711, entitled "Anatomical Modeling from a 3-D Image and a Surface Mapping," issued November 27, 2012, the disclosure of which is incorporated by reference herein; U.S. Pat. No. 7,720,521, entitled "Methods and Devices for Performing Procedures within the Ear, Nose, Throat and Paranasal Sinuses," issued May 18, 2010, the disclosure of which is incorporated by reference herein; U.S. Pat. Pub. No. 2014/0364725, entitled "Systems and Methods for Performing Image Guided Procedures within the Ear, Nose, Throat and Paranasal Sinuses," published December 11, 2014, the disclosure of which is incorporated by reference herein; U.S. Pub. No. 2016/0310042, entitled "System and Method to Map Structures of Nasal Cavity," published October 27, 2016; and U.S. Pat. Pub. No. 2011/0060214, entitled "Systems and Methods for Performing Image Guided Procedures within the Ear, Nose, Throat and Paranasal Sinuses," published March 10, 2011, the disclosure of which is incorporated by reference herein.

IGS navigation system (100) of the present example comprises a field generator assembly (200), which comprises set of magnetic field generators (206) that are integrated into a horseshoe-shaped frame (204). Field generators (206) are operable to generate alternating magnetic fields of different frequencies around the head of the patient. Field generators (206) thereby enable tracking of the position of a navigation guidewire (130) that is inserted into the head of the patient. Various suitable components that may be used to form and drive field generators (206) will be apparent to those of ordinary skill in the art in view of the teachings herein.

In the present example, frame (204) is mounted to a chair (300), with the patient (P) being seated in the chair (300) such that frame (204) is located adjacent to the head (H) of the patient (P). By way of example only, chair (300) and/or field generator assembly (200) may be configured and operable in accordance with at least some of the teachings of U.S. Patent App. No. 62/555,824, entitled "Apparatus to Secure Field Generating Device to Chair," filed September 8, 2017, the disclosure of which is incorporated by reference herein.

IGS navigation system (100) of the present example further comprises a processor (110), which controls field generators (206) and other elements of IGS navigation system (100). For instance, processor (110) is operable to drive field generators (206) to generate alternating current electromagnetic fields; and process signals from navigation guidewire (130) to determine the location of a sensor in navigation guidewire (130) within the head (H) of the patient (P). Processor (110) comprises a processing unit communicating with one or more memories. Processor (110) of the present example is mounted in a console (116), which comprises operating controls (112) that include a keypad and/or a pointing device such as a mouse or trackball. A physician uses operating controls (112) to interact with processor (110) while performing the surgical procedure.

A coupling unit (132) is secured to the proximal end of a navigation guidewire (130). Coupling unit (132) of this example is configured to provide wireless communication of data and other signals between console (116) and navigation guidewire (130). While coupling unit (132) of the present example couples with console (116) wirelessly, some other versions may provide wired coupling between coupling unit (132) and console (116). Various other suitable features and functionality that may be incorporated into coupling unit (132) will be apparent to those of ordinary skill in the art in view of the teachings herein.

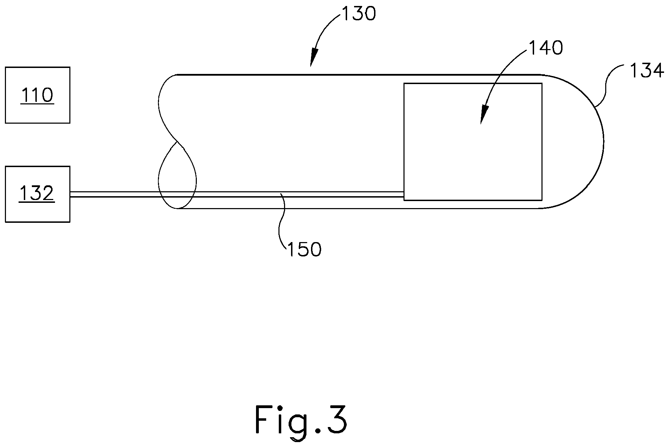

Navigation guidewire (130) may be used as a substitute for guidewire (30) in dilation instrument (20) described above. As shown in FIG. 3, navigation guidewire (130) includes a sensor (140) that is located at distal end (134) of navigation guidewire (130). Sensor (140) is in communication with coupling unit (132) via one or more wires (150) extending along the length of navigation guidewire (130). Sensor (140) is responsive to movement within the fields generated by field generators (206). In the present example, the sensor of navigation guidewire (130) comprises at least one coil at distal end (134) of navigation guidewire (130). When such a coil is positioned within an alternating current electromagnetic field generated by field generators (206), positioning of the coil within that magnetic field may generate electrical current in the coil, and this electrical current may be communicated along the electrical conduit(s) in navigation guidewire (130) and further to processor (110) via coupling unit (132). This phenomenon may enable IGS navigation system (100) to determine the location of the distal end (134) of navigation guidewire (130) within a three-dimensional space (i.e., within the head (H) of the patient (P)). To accomplish this, processor (110) executes an algorithm to calculate location coordinates of the distal end (134) of navigation guidewire (130) from the position related signals of the coil(s) in navigation guidewire (130).

Processor (110) uses software stored in a memory of processor (110) to calibrate and operate system (100). Such operation includes driving field generators (206), processing data from navigation guidewire (130), processing data from operating controls (112), and driving display screen (114). Processor (110) is further operable to provide video in real time via display screen (114), showing the position of distal end (134) of navigation guidewire (130) in relation to a video camera image of the patient's head (H), a CT scan image of the patient's head (H), and/or a computer generated three-dimensional model of the anatomy within and adjacent to the patient's nasal cavity. Display screen (114) may display such images simultaneously and/or superimposed on each other during the surgical procedure. Such displayed images may also include graphical representations of instruments that are inserted in the patient's head (H), such as navigation guidewire (130), such that the operator may view the virtual rendering of the instrument at its actual location in real time. By way of example only, display screen (114) may provide images in accordance with at least some of the teachings of U.S. Pub. No. 2016/0008083, entitled "Guidewire Navigation for Sinuplasty," published January 14, 2016, the disclosure of which is incorporated by reference herein. In the event that the operator is also using an endoscope, the endoscopic image may also be provided on display screen (114).

The images provided through display screen (114) may help guide the operator in maneuvering and otherwise manipulating instruments within the patient's head. When used as a substitute for guidewire (30) in dilation instrument assembly (10), navigation guidewire (130) may facilitate navigation of instrumentation of dilation instrument assembly (10) within the patient during performance of a procedure to dilate the ostium of a paranasal sinus; or to dilate some other anatomical passageway (e.g., within the ear, nose, or throat, etc.). It should also be understood that other components of dilation instrument assembly (10) may incorporate a sensor like the sensor of navigation guidewire (130), including but not limited to dilation catheter (40).

### III. Exemplary Sensor Coil Configurations

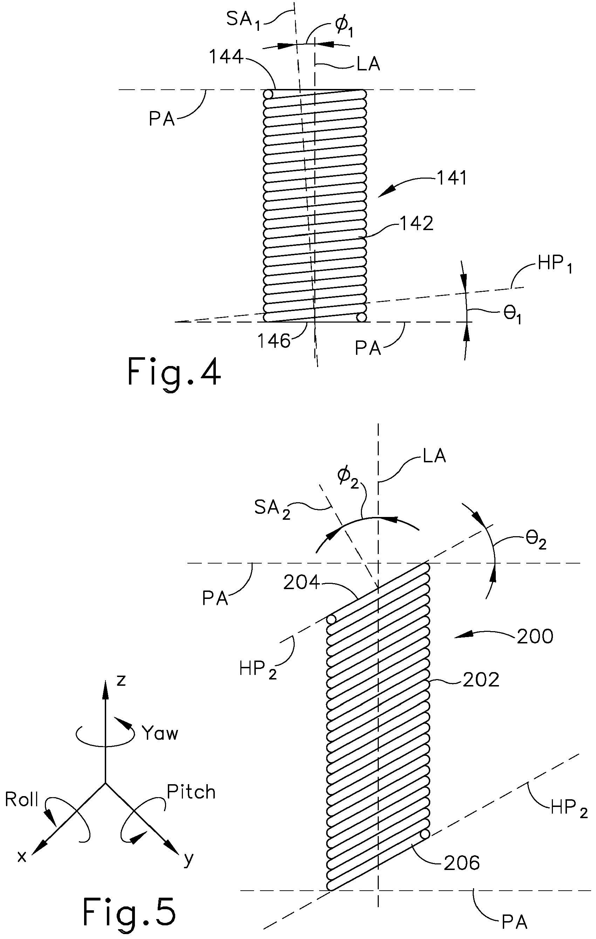

As noted above, sensor (140) of navigation guidewire (130) may take the form of a coil. FIG. 4 shows sensor (140) in the form of an exemplary coil (141). In this example, coil (141) is formed by a single, electrically conductive wire (142) that is wound in helical configuration. In other words, coil (141) is formed from a plurality of wire loops that are tightly stacked on top of one another and all are part of the same conductive wire (142). The helical configuration of coil (141) is longitudinally compressed with a fine pitch, in that each winding ofwire (142) is in contact with the adjacent windings of wire (142). Coil (141) is centered along a central longitudinal axis (LA). FIG. 4 also shows a pair of perpendicular axes (PA), which are perpendicular to the longitudinal axis (LA), and which are positioned at each longitudinal end of coil (141). The full length of coil (141) thus extends between these perpendicular axes (PA). The plane of each loop or winding is perpendicular to longitudinal axis (LA) and the center of each loop or winding falls on the longitudinal axis (LA). Each terminal end winding (144, 146) of coil (141) generally extends along a respective plane that is perpendicular to longitudinal axis (LA) in this example, such that each terminal end winding (144, 146) is generally aligned with a respective perpendicular axis (PA).

FIG. 4 also shows a helix plane (HP₁), which is defined as the plane indicating the orientation of each helical winding of wire (142). In other words, each winding is oriented along a respective helix plane (HP₁), with all the helix axes (HA₁) of coil (141) being parallel with each other. Helix plane (HP₁) of coil (141) defines a helix angle (θ₁) with perpendicular axis (PA). In this example, helix angle (θ₁) is relatively small (e.g., less than approximately 1°). A sensor axis (SA₁) extends perpendicularly from helix plane (HP₁). Sensor axis (SA₁) defines a sensor angle (ϕ₁) with longitudinal axis (LA). Sensor angle (ϕ₁) is equal to helix angle (θ₁), such that sensor angle (ϕ₁) is relatively small (e.g., less than approximately 1°).

By way of example only, coil (141) may be formed by wrapping wire (142) about a mandrel (not shown). The mandrel may comprise a cylindrical body that has a longitudinal axis aligned with longitudinal axis (LA) of the ultimately formed coil (141). Wire (142) is then wrapped around a winding axis that is parallel with the sensor axis (SA₁) of the ultimately formed coil (141). Since the sensor angle (ϕ₁) is relatively small, the winding axis is substantially aligned with the longitudinal axis of the mandrel.

As noted above, positioning of coil (141) within an alternating current electromagnetic field generated by field generators (206) will induce an electrical current in coil (141); and processor (110) may receive and process this electrical current to determine the location of the distal end (134) of navigation guidewire (130) within a three-dimensional space (i.e., within the head (H) of the patient (P)). In the context of a cartesian coordinate system, processor (110) may determine the x, y, and z coordinates of distal end (134) of guidewire (130) relative to a reference origin. However, with sensor angle (ϕ₁) being relatively small, it may be very difficult if not impossible for processor (110) to adequately determine the orientation of distal end (134) within three-dimensional space about three axes. In other words, while processor (110) may be able to determine that distal end (134) is positioned near a maxillary sinus ostium within the head (H) of the patient (P), processor (110) may be unable to determine whether the longitudinal axis (LA) of sensor (130) is oriented along the center of the maxillary sinus ostium or if the longitudinal axis (LA) of coil (141) of sensor (140) is oriented toward some anatomical structure that happens to be near the maxillary sinus ostium. For instance, as coil (141) is rotated about the longitudinal axis (LA) in a yaw movement, this yaw movement may induce a virtually imperceptible change in electrical current in coil (141), such that processor (110) may be unable to effectively detect the yaw movement, due to the relatively small sensor angle (ϕ₁), even if processor (110) is able to detect pitch and roll movements of coil (141).

It may be useful for the operator to know the orientation of distal end (134), in addition to knowing the position of distal end (134), in three-dimensional space. This orientation information may be particularly where distal end (134) of guidewire (130) includes a preformed bend (with sensor (140) being distal to the preformed bend). Conventional approaches may lead a person skilled in the art to include one or more additional coils in sensor (140) to provide orientation sensing capabilities to sensor (140). In particular, a conventionally constructed orientation sensitive sensor (140) may include a first coil configured and oriented like coil (141); and a second coil that is configured like coil (141) but oriented along a different longitudinal axis (LA) that is non-parallel with the longitudinal axis (LA) of the first coil (141). This additional coil may enable processor (110) to determine the orientation of the portion of the instrument in which the coils are integrated, in addition to being able to determine the position of the portion of the instrument in which the coils are integrated. However, this conventional approach may be undesirable for at least two reasons. First, having two or more coils may add to the complexity and/or cost of an instrument in which the coils are incorporated. Second, having two or more coils may make it difficult to reduce the form factor of the portion of the instrument in which the coils are integrated.

It may therefore be desirable to provide a version of sensor (140) that provides the enhanced orientation sensitivity that a conventional multi-coil sensor arrangement may provide; while avoiding the potential drawbacks of complexity and form factor that may be imposed by a multi-coil sensor arrangement. In particular, it may be desirable to provide a version of sensor (140) that provides the enhanced orientation sensitivity that a conventional multi-coil sensor arrangement may provide, yet only using a single coil instead of two or more coils.

FIG. 5 shows an exemplary alternative coil (200) that may be incorporated into sensor (140) in place of coil (141) in order to provide enhanced orientation sensitivity to sensor (140). Coil (200) of this example is formed by a single, electrically conductive wire (202) that is wound in helical configuration. The helical configuration of coil (200) is longitudinally compressed with a fine pitch, in that each winding of wire (202) is in contact with the adjacent windings of wire (202). Coil (200) is centered along a central longitudinal axis (LA). FIG. 5 also shows a pair of perpendicular axes (PA), which are perpendicular to the longitudinal axis (LA), and which are positioned at each longitudinal end of coil (200). The full length of coil (200) thus extends between these perpendicular axes (PA).

Each terminal end winding (204, 206) of coil (200) generally extends along a respective plane that is obliquely oriented relative to longitudinal axis (LA) and the respective perpendicular axes (PA). Instead, each terminal end winding (204, 206) of coil (200) generally extends along a helix plane (HP₂), which is defined as the axis indicating the orientation of each helical winding of wire (202). In other words, each winding is oriented along a respective helix plane (HP₂), with all the helix axes (HA₂) of coil (200) being parallel with each other. Helix plane (HP₂) of coil (200) defines a helix angle (θ₂) with perpendicular axis (PA). In this example, helix angle (θ₂) is substantially larger than helix angle (θ₁) (e.g., at least approximately 5°; between approximately 5° and approximately 45°; or more particularly about 20°). A sensor axis (SA₂) extends perpendicularly from helix plane (HP₂). Sensor axis (SA₂) defines a sensor angle (ϕ₂) with longitudinal axis (LA). Sensor angle (ϕ₂) is equal to helix angle (θ₂), such that sensor angle (ϕ₂) is substantially larger than sensor angle (ϕ₁) (e.g., at least approximately 5°; between approximately 5° and approximately 45°; or more particularly about 20°). The exemplary values and ranges of values for angles (θ₂, ϕ₂) provided above are merely illustrative examples and are not intended to be necessarily limiting.

By way of example only, coil (200) may be formed by wrapping wire (202) about a mandrel (not shown). The mandrel may comprise a cylindrical body that has a longitudinal axis aligned with longitudinal axis (LA) of the ultimately formed coil (200). Wire (202) is then wrapped around a winding axis that is parallel with the sensor axis (SA₂) of the ultimately formed coil (200). Since the sensor angle (ϕ₂) is relatively large, the winding axis is substantially non-parallel with the longitudinal axis of the mandrel.

As noted above, positioning of coil (200) within an alternating current electromagnetic field generated by field generators (206) will induce an electrical current in coil (200); and processor (110) may receive and process this electrical current to determine the location of the distal end (134) of navigation guidewire (130) within a three-dimensional space (i.e., within the head (H) of the patient (P)). In the context of a cartesian coordinate system, processor (110) may determine the x, y, and z coordinates of distal end (134) of guidewire (130) relative to a reference origin. Moreover, with sensor angle (ϕ₂) being relatively large, processor (110) may also determine the orientation of distal end (134) within three-dimensional space with relative ease (particularly as compared with data from coil (141)). In other words, processor (110) may be able to determine that distal end (134) is positioned near a maxillary sinus ostium within the head (H) of the patient (P); and whether the longitudinal axis (LA) of sensor (130) is oriented along the center of the maxillary sinus ostium or if the longitudinal axis (LA) of coil (200) of sensor (140) is oriented toward some anatomical structure that happens to be near the maxillary sinus ostium. Thus, as coil (200) is rotated about the longitudinal axis (LA) in a yaw movement, this yaw movement may induce a substantially perceptible change in electrical current in coil (200), such that processor (110) may be able to effectively detect the yaw movement, due to the relatively large sensor angle (ϕ₂).

In view of the foregoing, a sensor (140) incorporating just one single coil (200) may have the orientation sensitivity that might otherwise be associated with a conventional multi-coil sensor; while still maintaining the simplicity and relatively small form factor that is associated with a conventional single coil sensor. While coil (200) is described herein as being incorporated in a sensor (140) in a guidewire (130), a sensor (140) having just one single coil (200) may be incorporated in various other kinds of instruments. By way of example only, a sensor (140) having just one single coil (200) may be incorporated into curettes, picks, rosens, endoscopes, and/or various other kinds of instruments. Other kinds of instruments that may incorporate a sensor (140) having just one single coil (200) will be apparent to those of ordinary skill in the art in view of the teachings herein.

### IV. Exemplary Combinations

The following examples relate to various non-exhaustive ways in which the teachings herein may be combined or applied. It should be understood that the following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### Example 1

An apparatus comprising: (a) an elongate body, wherein the elongate body has: (i) a proximal end, and (ii) a distal end, wherein the distal end is sized and configured to fit through a naturally occurring orifice of a human body; and (b) a sensor positioned at the distal end of the elongate body, wherein the sensor comprises an electrically conductive coil, wherein the coil extends along a longitudinal axis, wherein the coil has a distal terminal winding and a proximal terminal winding, wherein the distal terminal winding extends along a plane that is obliquely oriented relative to the longitudinal axis, wherein the proximal terminal winding extends along a plane that is obliquely oriented relative to the longitudinal axis.

### Example 2

The apparatus of Example 1, wherein the coil defines a helix axis, wherein the helix axis defines a sensor angle with the longitudinal axis of the coil, wherein the sensor angle is at least approximately 5°.

### Example 3

The apparatus of any one or more of Examples 1 through 2, wherein the distal terminal winding extends along a plane that is oriented at an angle of at least approximately 5° relative to the longitudinal axis, wherein the proximal terminal winding extends along a plane that is oriented at an angle of at least approximately 5° relative to the longitudinal axis.

### Example 4

The apparatus of any one or more of Examples 1 through 3, wherein the sensor has only the electrically conductive coil, such that the sensor lacks any additional electrically conductive coils.

### Example 5

The apparatus of Example 4, wherein the elongate body has only the sensor with the single electrically conductive coil, such that the elongate body lacks any additional electrically conductive coils.

### Example 6

The apparatus of any one or more of Examples 1 through 5, wherein the distal end of the elongate body defines a longitudinal axis, wherein the longitudinal axis of the sensor is coextensive with the longitudinal axis of the distal end of the elongate body.

### Example 7

The apparatus of any one or more of Examples 1 through 6, wherein the elongate body is part of a guidewire.

### Example 8

The apparatus of any one or more of Examples 1 through 7, wherein the elongate body is part of an instrument selected from the group consisting of curettes, picks, rosens, and endoscopes.

### Example 9

The apparatus of any one or more of Examples 1 through 8, further comprising an image guided navigation system, wherein the image guided navigation system includes a processor, wherein the processor is configured to process a signal from electrical current generated by the coil and thereby determine a position of the coil in three-dimensional space.

### Example 10

The apparatus of Example 9, wherein the processor is further configured to process a signal from electrical current generated by the coil and thereby determine an orientation of the coil in three-dimensional space.

### Example 11

The apparatus of Example 10, wherein the processor is further configured to process a signal from electrical current generated by the coil and thereby determine a yaw position of the coil about the longitudinal axis.

### Example 12

The apparatus of any one or more of Examples 9 through 11, wherein the image guided navigation system further comprises one or more field generators operable to generate an alternating current electromagnetic field, wherein the coil is configured to generate the electrical current in response to positioning of the coil within an alternating current electromagnetic field generated by the one or more field generators.

### Example 13

The apparatus of any one or more of Examples 1 through 12, wherein the distal end of the elongate body, including the sensor, is sized and configured to fit through a nostril of a nose of a human body.

### Example 14

The apparatus of Example 13, wherein the distal end of the elongate body, including the sensor, is further sized and configured to fit through a paranasal sinus ostium of a human body.

### Example 15

The apparatus of any one or more of Examples 1 through 14, further comprising:
(a) a connector at the proximal end of the body, wherein the connector is configured to couple with an image guided navigation system; and (b) a wire extending along the elongate body, wherein the sensor is in communication with the connector via the wire.

### Example 16

An apparatus comprising: (a) an elongate body, wherein the elongate body has: (i) a proximal end, and (ii) a distal end, wherein the distal end is sized and configured to fit through a naturally occurring orifice of a human body; and (b) a sensor positioned at the distal end of the elongate body, wherein the sensor consists of just one single electrically conductive coil, wherein the coil extends along a longitudinal axis, wherein the coil has windings extending along respective helix axes, wherein each helix axis has a perpendicular sensor axis, wherein the sensor axis defines a sensor angle with the longitudinal axis, wherein the sensor angle at least approximately 5°.

### Example 17

The apparatus of Example 16, wherein the coil has a distal terminal winding and a proximal terminal winding, wherein the distal terminal winding extends along a plane that is obliquely oriented relative to the longitudinal axis, wherein the proximal terminal winding extends along a plane that is obliquely oriented relative to the longitudinal axis.

### Example 18

A method comprising: (a) inserting a distal end of an elongate instrument body into a patient, wherein the distal end of the elongate instrument body includes a sensor, wherein the sensor comprises a coil defining a longitudinal axis, wherein the coil has windings about the longitudinal axis, wherein the windings define a sensor axis, wherein the sensor axis defines a sensor angle with the longitudinal axis, wherein the sensor angle at least approximately 5°; (b) generating an alternating current electromagnetic field around a portion of the patient associated with the location of the distal end of the elongate instrument body; (c) moving the distal end of the elongate instrument body along the longitudinal axis in the patient, thereby moving the coil in the alternating current electromagnetic field, wherein the positioning of the coil in the alternating current electromagnetic field induces an electrical current in the coil, wherein an image guided navigation system processes the electrical current to determine a three-dimensional location of the distal end of the elongate instrument body in the patient; and (d) rotating the distal end of the elongate instrument body about the longitudinal axis, thereby rotating the coil in the alternating current electromagnetic field, wherein the rotation of the coil in the alternating current electromagnetic field induces an electrical current in the coil, wherein the image guided navigation system processes the electrical current to determine an orientation of the distal end of the elongate instrument body in the patient.

### Example 19

The method of Example 18, wherein the act of inserting a distal end of an elongate instrument body into a patient comprises inserting a distal end of an elongate instrument body into a nostril of a patient to thereby position the distal end in a nasal cavity of the patient.

### Example 20

The method of Example 19, further comprising: (a) advancing a dilation catheter along the elongate instrument body and into the nasal cavity of the patient; and (b) expanding a dilator of the dilation catheter to thereby dilate an anatomical passageway associated with the nasal cavity of the patient.

### V. Miscellaneous

It should be understood that any of the examples described herein may include various other features in addition to or in lieu of those described above. By way of example only, any of the examples described herein may also include one or more of the various features disclosed in any of the various references that are incorporated by reference herein.

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Versions of the devices disclosed herein can be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, versions of the device may be disassembled, and any number of the particular pieces or parts of the device may be selectively replaced or removed in any combination. Upon cleaning and/or replacement of particular parts, versions of the device may be reassembled for subsequent use either at a reconditioning facility, or by a surgical team immediately prior to a surgical procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be processed before surgery. First, a new or used instrument may be obtained and if necessary cleaned. The instrument may then be sterilized. In one sterilization technique, the instrument is placed in a closed and sealed container, such as a plastic or TYVEK bag. The container and instrument may then be placed in a field of radiation that can penetrate the container, such as gamma radiation, x-rays, or high-energy electrons. The radiation may kill bacteria on the instrument and in the container. The sterilized instrument may then be stored in the sterile container. The sealed container may keep the instrument sterile until it is opened in a surgical facility. A device may also be sterilized using any other technique known in the art, including but not limited to beta or gamma radiation, ethylene oxide, or steam.

Having shown and described various versions of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, versions, geometries, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. An apparatus comprising:
(a) an elongate body, wherein the elongate body has:
(i) a proximal end, and
(ii) a distal end, wherein the distal end is sized and configured to fit through a naturally occurring orifice of a human body; and
(b) a sensor positioned at the distal end of the elongate body, wherein the sensor comprises an electrically conductive coil, wherein the coil extends along a longitudinal axis, wherein the coil has a distal terminal winding and a proximal terminal winding,
wherein the distal terminal winding extends along a plane that is obliquely oriented relative to the longitudinal axis,
wherein the proximal terminal winding extends along a plane that is obliquely oriented relative to the longitudinal axis.

2. The apparatus of claim 1, wherein the coil defines a helix axis, wherein the helix axis defines a sensor angle with the longitudinal axis of the coil, wherein the sensor angle is at least approximately 5°.

3. The apparatus of claim 1, wherein the distal terminal winding extends along a plane that is oriented at an angle of at least approximately 5° relative to the longitudinal axis, wherein the proximal terminal winding extends along a plane that is oriented at an angle of at least approximately 5° relative to the longitudinal axis.

4. The apparatus of claim 1, wherein the sensor has only the electrically conductive coil, such that the sensor lacks any additional electrically conductive coils.

5. The apparatus of claim 4, wherein the elongate body has only the sensor with the single electrically conductive coil, such that the elongate body lacks any additional electrically conductive coils.

6. The apparatus of claim 1, wherein the distal end of the elongate body defines a longitudinal axis, wherein the longitudinal axis of the sensor is coextensive with the longitudinal axis of the distal end of the elongate body.

7. The apparatus of claim 1, wherein the elongate body is part of (i) a guidewire, or (ii) an instrument selected from the group consisting of curettes, picks, rosens, and endoscopes.

8. The apparatus of claim 1, further comprising an image guided navigation system, wherein the image guided navigation system includes a processor, wherein the processor is configured to process a signal from electrical current generated by the coil and thereby determine a position of the coil in three-dimensional space.

9. The apparatus of claim 8, wherein the processor is further configured to process a signal from electrical current generated by the coil and thereby determine an orientation of the coil in three-dimensional space.

10. The apparatus of claim 9, wherein the processor is further configured to process a signal from electrical current generated by the coil and thereby determine a yaw position of the coil about the longitudinal axis.

11. The apparatus of claim 8, wherein the image guided navigation system further comprises one or more field generators operable to generate an alternating current electromagnetic field, wherein the coil is configured to generate the electrical current in response to positioning of the coil within an alternating current electromagnetic field generated by the one or more field generators.

12. The apparatus of claim 1, wherein the distal end of the elongate body, including the sensor, is sized and configured to fit through a nostril of a nose of a human body.

13. The apparatus of claim 12, wherein the distal end of the elongate body, including the sensor, is further sized and configured to fit through a paranasal sinus ostium of a human body.

14. The apparatus of claim 1, further comprising:
(a) a connector at the proximal end of the body, wherein the connector is configured to couple with an image guided navigation system; and
(b) a wire extending along the elongate body, wherein the sensor is in communication with the connector via the wire.

15. An apparatus comprising:
(a) an elongate body, wherein the elongate body has:
(i) a proximal end, and
(ii) a distal end, wherein the distal end is sized and configured to fit through a naturally occurring orifice of a human body; and
(b) a sensor positioned at the distal end of the elongate body, wherein the sensor consists of just one single electrically conductive coil, wherein the coil extends along a longitudinal axis, wherein the coil has windings extending along respective helix axes, wherein each helix axis has a perpendicular sensor axis, wherein the sensor axis defines a sensor angle with the longitudinal axis, wherein the sensor angle at least approximately 5°.

16. The apparatus of claim 15, wherein the coil has a distal terminal winding and a proximal terminal winding,
wherein the distal terminal winding extends along a plane that is obliquely oriented relative to the longitudinal axis,
wherein the proximal terminal winding extends along a plane that is obliquely oriented relative to the longitudinal axis.
